# EUROPEAN PATENT APPLICATION

(11) **EP 2 388 016 A1**
(43) Date of publication of application: **23.11.2011**
(21) Application number: 11167186.3
(22) Date of filing: 23.05.2011
(51) Int. Cl.: A61K 47/48, C12N 15/867

(54) **Non-viral nucleic acid molecules delivery systems**

(30) Priority: 21.05.2010 IT TO20100428
(71) Applicant: Ferruti, Paolo, 20145 Milano (IT); Cavalli, Roberto, 15100 Alessandria (IT); Ranucci, Elisabetta, 20090 Opera (IT); Lembo, David, 10137 Torino (IT); Trotta, Francesco, 14100 Asti (IT); Primo, Luca, 10064 Pinerolo (IT)
(72) Inventor: The designation of the inventor has not yet been filed
(74) Representative: Jorio, Paolo

(57) **Abstract**

Use of an agmatine-based poly(amidoamine) polymer of formula (I) 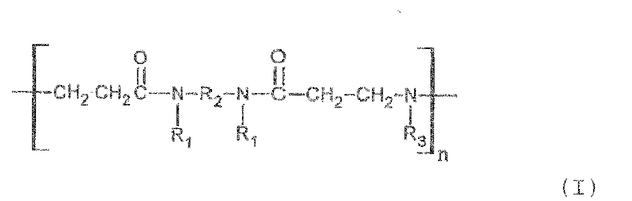 wherein
n is an integer in the range between 20 and 70;
R¹ is H, C₁₋₆ alkyl; or taken together with the N atoms to which is bound and R² is a piperazine ring;
R² is C₂₋₆ alkylen; C₅₋₆ cycloalkylen; CH-COOH, CH-COOR,
wherein R is C₁₋₄ alkyl, phenyl or benzyl;
R³ is -(CH₂)ₚ-HN-C(NH₂)=NH,
p is an integer from 1 to 6,
wherein said poly(amidoamine) polymer has a number average molecular weight in the range between 3300 and 33000 and a polydispersity index in the range between 1.25 and 1.75 as an *in vitro* and/or *in vivo* delivery system of at least one RNA molecule into a cell.

## Description

### Field of the invention

This disclosure concerns non-viral nucleic acid molecules delivery systems.

### Background of the invention

In the last decades, several systems, including viral and non-viral carriers, have been developed to transfer foreign genetic materials into cells with the aim of enhancing gene transfer. Despite the progress in biotechnology and pharmaceutical technology, a major problem in exploiting the full potential of gene therapy is the lack of safe and efficient delivery systems for *in vivo* application. As viral vectors possess toxicity and immunogenicity, non-viral strategies are becoming more and more attractive. Synthetic non-viral vectors are potentially less immunogenic, can be tailored to interact specifically with nucleic acids and might be delivered to specific tissues by incorporation of a targeting ligand.

There has been an increased interest in cationic lipids and polymers as nucleic acid condensing agent for gene delivery. However, many of them, such as for instance polylysine (PLL), polyethylenimine (PEI) and polyamidoamine (PAMAM) dendrimers, are toxic and can hardly be considered for future *in vivo* application. Other potential carriers, such as for instance poly(β-aminoester)s, α,β-poly(asparthylhydrazide)-glycidyl-trimethylammoniumchloride copolymers and polyethylenimine-alt-poly(ethylene glycol) copolymers, exhibit better safety profiles, but their efficiency is low and if injected are rapidly captured by the reticulo-endothelial system (RES) cells.

Poly(amidoamine)s (PAAs) are synthetic biodegradable polymers that can be designed to be highly biocompatible. They were first described in 1970 [1] and, subsequently, their physico-chemical and biological properties were reviewed at intervals [2-4]. PAAs are obtained by stepwise Michael-type polyaddition of primary or secondary amines to *bis*-acrylamides, and carry amide (a) and ter-amine (b) groups regularly arranged along the polymer chain in sequence either a.a.b.b or a.a.b, according to the type of amine monomer employed. Many PAAs exhibit a combination of properties imparting them a considerable potential in the biomedical field. They are degradable in water at a rate depending on their structure. Therefore, if injected, they are bioeliminable. PAAs are usually water-soluble and all of them, including amphoteric ones carrying carboxyl groups as side substituents, show different charge distribution profiles as a function of pH. As polycations, PAAs are remarkably cytobiocompatible. The toxicity of PAAs is a function of their basic strength, the most basic ones being the most toxic, but by far the majority of them exhibit LD50 values *in vitro* higher by two orders of magnitude or more than PLL, PEI or PAMAM dendrimers. Amphoteric PAAs are even less toxic, some of them being approximately as biocompatible as dextran. The haemolytic activity of PAAs follows the same trend. The ability of linear PAAs to give complexes with DMA and to promote transfection *in vitro* was first demonstrated for some non-amphoteric PAAs. A linear PAA of new generation, nicknamed AGMA1, obtained by polyaddition of 4-aminobutylguanidine (that is, agmatine) to 2,2-bisacrylamidoacetic acid was also studied in this respect [5]. AGMA1 is amphoteric, but prevailingly cationic and represents a considerable improvement over all other PAAs previously studied as DNA carriers in that it is prevailingly cationic up to pH 10.5 or whereabouts (for instance, at the blood pH of 7.4 it carries on the average 0.55 positive charges per repeating unit), yet it is highly cell biocompatible and, moreover, it is not haemolytic, that is, is not membrane-destabilising at any reasonable physiological pH. AGMA1, unlike any other polycation studied so far, after injection in animals circulates for a long time in the blood stream, a rare behaviour previously found for some neutral hydrophilic polymers or, as regards PAAs, some amphoteric, but prevailingly anionic ones. In fact, AGMA1 appears endowed with a unique combination of properties among amino polymers and warrants a definite potential as DNA carrier not only *in vitro* but also *in vivo*.

The still unresolved technical problems connected with the use of all the other non-viral nucleic acid carriers proposed in the literature (including the other previously studied PAAs), and especially their toxicity and liability to be rapidly wiped out *in vivo* by the body's defence systems, are in principle overcome by this agmatine-based PAA.

RNA interference-based specific gene silencing has high therapeutic potential and various synthetic carriers are in the developing stage. These include polymers, lipids and nanoparticles. The delivery systems should protect siRNA and enable cellular uptake and transport to the cytosol. One common approach is the use of delivery systems originally employed for DNA. Consequently different cationic polymers have been studied as siRNA carriers. PEI has been widely studied showing the capacity of delivering siRNA both *in vitro* and *in vivo.*

However, recent studies [8] have demonstrated the great structural differences existing between DNA and siRNA that do not render easy the application of any polymer used for DNA delivery also to siRNA delivery.

PEI has been widely studied showing the ability of relasing siRNA both *in vitro* and *in vivo*.

Bioreducible polymers represent carriers of choice for siRNA delivery due to their cytosol-specific degradation. SS-PAEI efficiently complexed siRNA and then release under the reductive milieu of cytosol.

A reducible poly(amido-ethylenimine) was recently used as a carrier for small interference RNA (siRNA).

However, all the amine polymers carriers tested so far present the same shortcomings outlined above when dealing with transfection promoters, especially when considered for *in vivo* applications. As regards liposomes and lipid nanoparticles the main problem is the stability of their siRNA combinations both in the shelf and in living organisms.

### Summary of the invention

Taking into account these premises, the need is therefore felt for improved solutions enabling the delivery of RNA molecules, like for example siRNA molecules, into the cells avoiding the above referenced disadvantages.

The object of this disclosure is providing such improved solutions.

According to the invention, the above object is achieved thanks to the subject matter recalled specifically in the ensuing claims, which are understood as forming an integral part of this disclosure.

An embodiment of the present disclosure provides the use of an agmatine-based poly (amidoamine) polymer of formula (I): 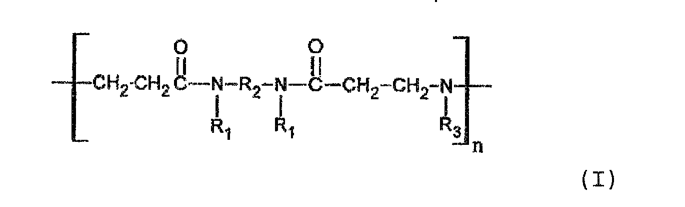 wherein n is an integer in the range between 20 and 70;
R¹ is H, C₁₋₆ alkyl; or taken together with the N atoms to which is bound and R² is a piperazine ring;
R² is C₂₋₆ alkylen; C₅₋₆ cycloalkylen; CH-COOH, CH-COOR, wherein R is C₁₋₄ alkyl, phenyl or benzyl;
R³ is -(CH₂)ₚ-HN-C(NH₂)=NH,
wherein p is an integer from 1 to 6,
as an *in vitro* and/or *in vivo* delivery system of at least one RNA molecule into a cell.

The agmatine-based poly(amido-amine) polymer of formula (I) has a molecular weight in the range between 3,300 and 33,000 Da and a polydispersity index in the range between 1.10 and 1.30. Such a delivery system can be - in a surprising manner - employed to deliver into cells RNA, siRNA molecules with high efficacy.

The poly(amidoamine) polymer of formula (I) is able to complex the RNA/siRNA molecule by forming nanoparticles having a size lower than 150 nm, preferably lower than 100 nm. The poly(amidoamine) polymer forms stable complexes with the RNA/siRNA molecule in a nucleic acid molecule:polymer weight ratio in the range between 1:3 and 1:50, preferably 1:10 and 1:40.

### Brief description of the drawings

The invention will now be described, by way of example only, with reference to the enclosed figures of drawing, wherein:
- **Figure 1****:** Chemical structure of AGMA1 poly(amidoamine) polymers.
- **Figure 2****:** Zeta potential of siRNA complexes with Agma1-24
- **Figure 3****:** TEM microphotograph of the complex:
   siRNA:AGMA1-10 (A), siRNA:AGMA1-24 (B), siRNA:AGMA1-50 (C) 1:20 w/w (Magnification 130000X).
- **Figure 4****:** Western blot analysis of lysates from Hela cells transfected with control or AKT-1 siRNA using AGMA1-24 or AGMA1-10 polymers as carriers. The siRNA efficiency of knock-down is detected by anti-Akt and anti-βactin antibodies.
- **Figure 5****:** Intracellular trafficking and nuclear localization of FITC-labelled AGMA1-24.

### Detailed description of the invention

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

### EXAMPLES

### EXAMPLE 1

Three AGMA1 samples, named AGMA1-10, AGMA1-24 and AGMA1-50, with different molecular weights (3300, 7800 and 16400) were synthesized as RNA/siRNA carriers and firstly characterized in terms of molecular weight, charge, cytotoxicity and haemolytic activity. The charge distribution profiles showed that they were amphoteric, but prevailingly cationic at all physiological pH values. The positive charge and the *in vitro* lack of cytotoxicity and haemolytic activity make AGMA1 samples valuable candidates as RNA/siRNA delivery systems. Broadly speaking, indeed, cationic polymers other than AGMA1 suffer of being toxic for the cells.

The three PAA samples studied showed the capacity of forming by electrostatic attraction polyplexes condensing siRNA into small nanometric particles with spherical shape. AGMA1-10, AGMA1-24 and AGMA1-50 were able to complex siRNA at different weight ratios and pH values. For instance, AGMA1-10, the lower molecular weight polymer, formed larger complexes than AGNA1-24 under the condition studied, probably due to a non complete binding and a weakly condensation of plasmid siRNA.

AGMA1-24 polyplexes showed sizes lower than 100 nm and the size value varied according to the complexation ratio. The average diameter of the polyplexes did not increase over time and after freezing maintained favorable size to be taken up by cells. It is generally considered that 150 nm represents a size limit for no-specific cell uptake. Lager particles of 200-300 nm are found to be efficient in gene transfer in cell cultures, but smaller siRNA particles are important for *in vivo* use.

The surface charge of these siRNA nanoparticles turned to be positive at physiological pH values favoring the electrostatic interactions between the gene carriers and the cell surface that are negatively charged for the presence of proteoglycans and sialylated glycoproteins.

For *in vitro* transfection, particles bearing positive charge facilitated the uptake by negative cell membranes. However *in vivo,* nanoparticles may encounter several negative charged macromolecules, like serum protein albumin, which can interact with them.

AGMA1-10, AGMA1-24 and AGMA1-50 were also able to condense siRNA in small nanoparticles of about 50 nm, size that can be easily internalized in cells and suitable for activity. It is recently reported that siRNA complexes greater than 150 nm were unable to mediate gene silencing.

Surface charge of siRNA nanoparticles zeta potential values ranging from + 16 mV to + 20 mV.

RNA complexation with AGMA1-10, AGMA1-24 and AGMA1-50 was able to protect siRNA against enzymatic degradation. The protection capacity confirmed the encapsulation of nucleic acids by the three polymers. For siRNA the protection against degradation by nucleases is of primary importance.

To establish the relationship between the PAA molecular weight, structure and their gene transfer ability the present inventors carried out transfection experiments on HeLa cells, a well characterized and difficult to transfect cell line. Hela cells represents moreover a model for molecular medicine studies.

A marked difference between AGMA1-10, AGMA1-24 and AGMA1-50 was evidenced. The ability of the AGMA1-24 and AGMA1-50 to act as a siRNA carrier was assessed using the gel retardation assay.

siRNA silencing capacity was observed *in vitro* with the AGMA1-24 complexes. AGMA1-24, and higher molecular weight agmatine derivatives, represent the proper proportion between molecular weight and charge density to achieve an optimal complexation of nucleic acids. AGMA1-24 and AGMA1-50 were found - unexpectedly - to successfully deliver siRNA. The AGMA1 molecular weight represents a critical characteristic for use as delivery system of nucleic acids into cells. Linear Agmatine-containg PAA with molecular weights higher than 6,000 Da satisfy the requirements to be considered as non-viral RNA/siRNA polymer carriers.

### MATERIALS AND METHODS

### Instruments

The ¹H and ¹³C spectra were acquired on a Brüker Avance 400 spectrometer, operating at 400.133 MHz (1H) and at 100.623 MHz (13C). Size exclusion chromatography (SEC) traces were obtained making use of TSK-gel G4000 PW and TSK-gel G3000 PW columns produced by TosoHaas. The two columns were connected in series, and the mobile phase was Tris buffer, pH 8.00, flow rate 1 mL/min (Waters model HPLC pump 515). The UV detector was a Waters model 486, operating at 230 nm. The refractive detector was a Waters model 2410. The samples were prepared in Tris buffer with a 1% w/v concentration is polymer. Molecular weight determinations were based on a Viskotek Laser Low Angle Light Scattering (LLALS) detector. Fluorimetric measurements were performed with an RF 551 Shimadzu fluorimeter.

### Materials

Ultra-pure water was obtained using a system 1-800 Milli-Q (Millipore, F). Fluoresceine isotiocianate (FITC) ethidium bromide and etoposide were purchased from Fluka (CH). Dnase II was supplied by Sigma (UK). siRNA duplex was purchased by Ambion Applied Biosystem (ID #s695). Agarose was purchased from BIO-RAD Laboratories S.r.1 (Milan). JetPEI was purchased from Polyplus-Transfection (Strasbourg, France). All other reagents (ACS grade) were from Sigma and were used as received. High-performance liquid chromatography (HPLC) solvents were from Carlo Erba (Italy). 2,2-Bisacrylamidoacetic acid (BAC) was prepared as previously described [6].

### Preparation of PAA polymers

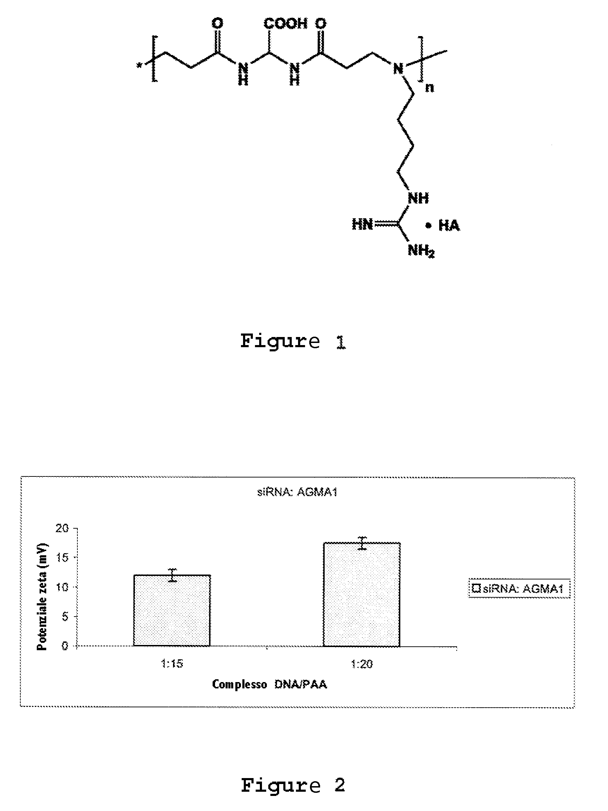

The three linear PAA samples, named AGMA1-10, AGMA1-24 and AGMA1-50 having the structure showed in figure 1, were prepared following a general method and recipe already described [5]. Briefly, Agmatine sulfate (2.000 g, 8.5 mmol) and lithium hydroxide monohydrate (0.360, 8.5 mmol) were added to a solution of BAC (1.689 g, 8.5 mmol) and lithium hydroxide monohydrate (0.360 g, 8.5 mmol) in distilled water (2.8 mL). This mixture was maintained under nitrogen atmosphere and occasionally stirred for 48 (AGMA1-10), 72 (AGMA1-24), or 240 (AGMA1-50) hrs. After this time, it was diluted with water (2.8 mL), acidified with hydrochloric acid to pH 4-4.5, and then ultrafiltered through membranes with nominal cut-off 3000 (AGMA1-10), 5000 (AGMA1-24) and 10000 (AGMA1-50), respectively. The fractions retained in each case were freeze-dried and the product obtained as a white powder. Yield: 2.1 g. ¹H NMR (D₂O) : δ (ppm)) 1.61 (br, NHCH₂CH₂CH₂), 1.76 (br, NHCH₂CH₂), 2.79 (br, NHCOCH₂CH₂), 3.19 (m, NHCH₂, NCH₂), 3.44 (br, NHCOCH₂), 5.55 (s, COOHCH). ¹³C NMR (D₂O): δ (ppm)) 22.3 (NHCH₂CH₂CH₂), 25.0 (NHCH₂CH₂), 28.9 (NHCOCH₂CH₂), 40.4 (CH₂NCH₂), 49.1 (NHCOCH₂), 52.5 (NHCH₂), 56.0 (COOHCH), 155.1 (NH₂CNNH), 171.3 (NHCO), 173.5 (CHCOOH). Molecular weight values were: AGMA1-10, *M̅n* 3300, *M̅w* 4500, d= 1.38; AGMA1-24, *M̅n* 7800, *M̅w* 10100 and d 1.29; AGMA1-50, *M̅n =* 16400, *M̅w*= 20500, d = 1.25.

### Preparation of fluorescent PAAs (FITC-AGMA1-24)

Labeled AGMA1-24 (FTTC-AGMA1-24) was prepared using AGMA1-24-NH₂ obtained as previously described [2] in aqueous solution at pH 7.4 (10 mg/mL) with a FITC solution in methanol (0.2 mg/mL). The resultant mixture was stirred overnight at room temperature and then centrifuged to eliminate insoluble impurities. The resultant clear solution was then dialyzed and the fluorescein-labeled polymer isolated by freeze-drying the retained portion. The recovery was practically quantitative. The conjugation of AGMA1-24 -NH2 with FITC was confirmed by NMR.

### Characterization of the polymers

### Zeta Potential determination

The Zeta-Potential values (PZ) of AGMA1-10, AGMA1-24 and AGMA1-50 were determined in aqueous solutions at increasing pH values, ranging from 4.0 to 7.4, to verify the polymer charge distribution as function of the pH. A 90 Plus instrument (Brookhaven, NY, USA) was used to determine the electrophoretic mobility and the zeta potential of the two polymers. For the determinations, the aqueous solutions of AGMA1-10, AGMA1-24 and AGMA1-50 were placed in the electrophoretic cell, where an electric field of about 15 V/cm was applied. Each value reported is the average of ten measurements. The electrophoretic mobility measured was converted into zeta potential using the Smoluchowsky equation [7].

### Biocompatibility assessment

Haemolytic activity of AGMA1-10, AGMA1-24 and AGMA1-50 was studied on human blood. Increasing amounts of the three polymers up to 15 mg/ml were added to a suspension of erythrocytes (30 % v/v) in phosphate buffer pH 7.4 and then incubated for 90 minutes at 37°C. A suspension containing only a 30 % v/v of erythrocytes in phosphate buffer pH 7.4 was used as blank. Another suspension added with an excess of ammonium chloride was used to obtain complete hemolysis as 100% hemolytic control. After centrifugation at 2000 rpm for 5 minutes the supernatants were analyzed using a Lambda 2 Perkin-Elmer spectrophotometer at a wavelength of 543 nm. The percentage of hemolysis was calculated versus the 100% hemolysis control. The cytoxicity of AGMA1-20, AGMA1-24 and AGMA1-50 were assessed on Hela cells seeded in 24 wells plate. The toxicity of the polymer was evaluated by MTT assay (MTT = 3-(4,5-Dimethylthiazol-2-yl)-2,5-diphenyltetrazolium bromide)). The measurement was performed at 24 and 48 hours post-incubation. The viability of the treated cells was compared to untreated cells and to cell treated with Etoposide (0.1 µg/µl), a toxic compound.

### Preparation and characterization of siRNA/PAA complexes

### Preparation of the complexes

To prepare siRIMA/PAA complexes different amount of a AGMA1-10 or AGMA1-24 or AGMA1-50 solution (1.0 mg/ml) in sodium chloride 0.9% w/v were added to siRNA solution (50µM) in sodium chloride 0.9% w/v to obtain different complex weight ratios (i.e 1:10, 1:20, 1:50). After mixing, the siRNA complexes were incubated for 30 minutes at room temperature before characterization and activity determination.

### Size, superficial charge and morphology of siRNA/PAA complexes

The average diameters and polydispersity indices of the siRNA/PAA complexes were determined by photocorrelation spectroscopy (PCS) using a 90 Plus instrument (Brookhaven, NY, USA) as previously described for DNA/PAA complexes. The electrophoretic mobility and zeta potential of the complexes were determined using the same 90 Plus instrument (Brookhaven, NY, USA) with the addition of an electrode as previously described for the characterization. The morphology of siRNA complexes with AGMA1-10, AGMA1-24 and AGMA1-50 was determined by Transmission Electron Microscopy (TEM). TEM analyses were carried out using a Philips CM10 instrument (Eindoven, NL). siRNA/PAA complexes in solution were dropped onto a Formwar-coated copper grid and air-dried before observation.

### in vitro siRNA transfection studies

### siRNA transfection experiments

Knockdown of AKT1 gene was performed using 21 bp siRNA. duplex against AKT-1 protein. The duplex sense sequence was: GCGUGACCAUGAACGAGUU (SEQ ID No.:1). Both siRNA and AGMA1 polymer were pre-incubated in a solution of NaCl 150 nM pH 5.5 for 30 min at room temperature with siRNA final concentration of 135 nM. Hela cells at 4.5 × 10⁵ cells per well were transiently transfected in 6 wells plate.

### Western blot experiments

Total proteins were extracted in Laemmli buffer (62.5 mM Tris-HCl pH 6.8, 2% SDS, 10% glycerol), quantified and equal amounts of each sample were resolved by SDS-PAGE and transferred to PVDF membrane. After blocking with TBS/ 0.1% Tween 20/ 5% BSA, membranes were incubated with primary antibody over-night at 4°C. Primary antibodies used are: α-Akt (Cell Signaling), α-GFP (Molecular Probes), α-tubulin and α-actin (Santa Cruz Biotechnology). Immunoreactive proteins were identified with secondary antibody coupled to horseradish peroxidase (HRP) antibody and visualized by ECL.

### RESULTS

### Chemistry and polymer characterization

Three linear agmatine-containing PAAs, AGMA1-10, AGMA1-24 and AGMA1-50, of different molecular weights and narrow distribution were synthesized as reported in "Materials and Methods" section, adopting three different reaction times to tune the molecular weight and studied as intracellular siRNA carriers. In particular, AGMA1-10, AGMA1-24 and AGMA1-50 had the following molecular weights: AGMA1-10, *M̅n* 3300, *M̅w* 4500, d= 1.38; AGMA1-24, *M̅n* 7800, *M̅w* 10100 and d 1.29; AGMA1-50, *M̅n =* 16400, *M̅w* = 20500, d = 1.25. It may be observed that the polydispersity values (*d*) of the polymers were fairly low, due to the purification procedure by membrane ultrafiltration adopted in their preparation. The conjugation of PAAs with FITC was confirmed by NMR, and the efficiency of the labelling procedure was determined by measuring the fluorescence intensity at λₑₓ=480 nm and λₑₘ=520 nm of a solution of FITC-PAA of known concentration versus a standard FITC solution. The viscosity and molecular weight values found for AGMA1-10, AGMA1-24 and AGMA1-50 conjugated with FITC were very similar to those previously determined for the three polymers showing that labelling did not induce any significant alteration of properties. Zeta potential measurements demonstrated that AGMA1-10, AGMA1-24 and AGMA1-50 are positively charged in aqueous solution at pH 7.4 and the positive charge increase lowering the pH to 4.0 (table 1).

**Table 1**

| **Polymer** | **Zeta Potential (mV)** | | | |
|---|---|---|---|---|
| | **pH 4.0** | **pH 5.0** | **pH 6.0** | **pH 7.4** |
| **AGMA1-10** | 32.68 ± 1.37 | 31.59 ± 0.43 | 22.59 ± 0.67 | 17.09 ± 0.60 |
| **AGMA1-24** | 21.00 ± 1.16 | 15.32 ± 0.74 | 11.10 ± 0.52 | 2.10 ± 0.37 |
| **AGMA1-50** | 20.05 ± 1.3 | 16.03 ± 0.82 | 10.30 ± 0.49 | 3.56 ± 0.77 |

### PAA biological properties

No significant haemolytic activity was observed for the three polymers after 90 minutes of incubation in blood at pH 7.4 up to a concentration of 15 mg/ml. Moreover the three polymers showed no cytotoxic effects on Hela cells after 48 hours of incubation at a concentration of 100 µg/ml.

### siRNA complexation

AGMA1-10, AGMA1-24 and AGMA1-50 are able to complex siRNA as confirmed by positive Zeta potential values at all the ratio studied. The sizes of siRNA/ACMA-10, AGMA-24 and AGMA1-50 complexes determined by dynamic light scattering were lower than 100 nm.

### Sizes siRNA/AGMA1-10, 24, 50

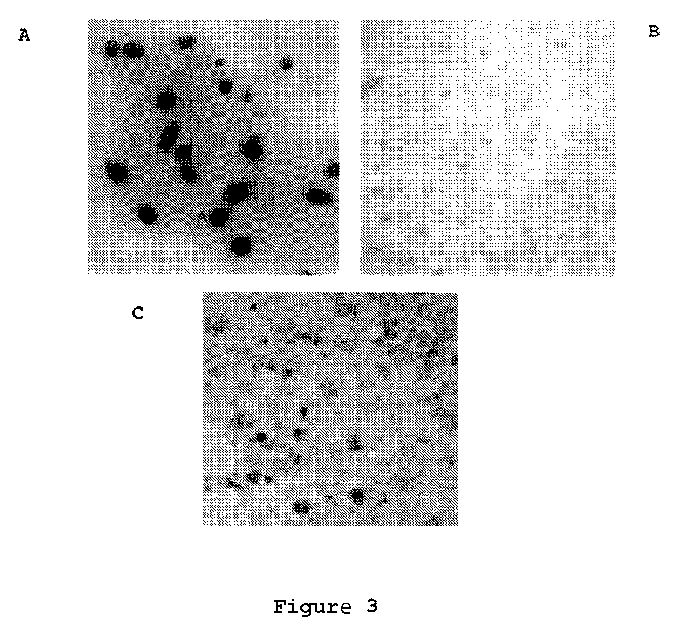

Direct observation of all the complexes by TEM shows that the polyplexes of siRNA are obtained in the form of discrete spherical nanoparticles. The siRNA/AGMA1-10, AGMA1-24 and AGMA1-50 complexes range from about 20 nm to 50 nm (Figure 3).

### Gene transfer efficacy and cytotoxicity of the complexes

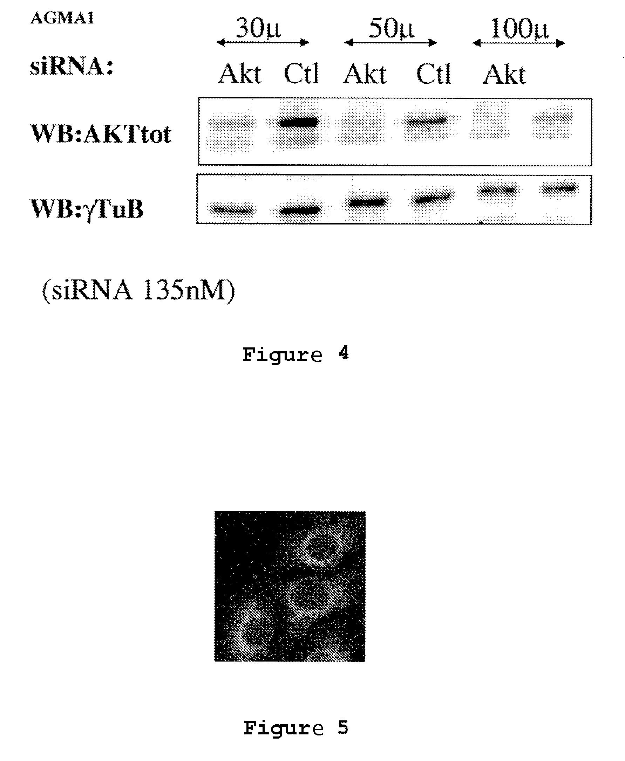

AGMA1-10 AGMA1-24 and AGMA1-50 were efficiently able to complex siRNA forming nanoparticles. The present inventors evaluated whether these complexes were also able to silence specific gene. When Hela cells were treated with AGMA1-24-complexed with Akti-specific siRNA, an evident (at least 50%) reduction of endogenous Akt1 protein expression compared with treatment with AGMA1-24 complexed with nonspecific siRNA (Fig. 4). The results obtained with the AGMA1-50/siKNA complexes were practically superimposible to those of AGMA1-24/siRNA complexes.

### Intracellular trafficking and nuclear localization

For this purpose the present inventors used FITC-labelled AGMA1-24 and AGMA1-50. Intracellular trafficking of FITC-labelled AGMA1-24 was analyzed by confocal microscopy and the result is shown in Figure 6. In order to display the intracellular localization of AGMA1-24, fluorescent phalloidin was used to stain cortical actin and stress fiber in red. AGMA1-24 and AGMA1-50 are almost totally sequestrated inside the cell just 30 min. after treatment, as it results evident from Figure 5.

### EXAMPLE 2

### MATERIALS AND METHODS

### siRNA transfection

Silencing of AKT1 gene was obtained using 21 bp siRNA duplex purchased by Ambion-Applied Biosystem (ID #s695) against AKT-1 protein. The duplex sense sequence was: GCGUGACCAUGAACGAG UUtt. Silencing of Rab5 was obtained with Dharmacon Pooled-siRNA anti-Rab5. Both siRNA and Rb polymer were pre-incubated in a solution of pH NaCl 150nM 5,5 for 30 min at room temperature with siRNA final concentration of 50 nM and 100nM. Hela and PC3 cells at 4,5x 10⁵ cells per well were transiently transfected in 6 wells plate.

### Western blot

Total proteins were extracted in Laemmli buffer (62.5 mM Tris-HCl pH 6.8, 2% SDS, 10% glycerol), quantified and equal amounts of each sample were resolved by SDS-PAGE and transferred to PVDF membrane. After blocking with TBS/ 0.1% Tween 2.0/ 5% BSA, membranes were incubated with primary antibody over-night at 4°C. Primary antibodies used are: α-Akt (Cell Signaling), α-Rab5 (Molecular Probes), α-tubulin and α-actin (Santa Cruz Biotechnology). Immunoreactive proteins were identified with secondary antibody coupled to horseradish peroxidase (HRP) antibody and visualized by ECL.

### Results

Since Rb10 and Rb5 were able to efficiently complex with siRNA, we evaluated whether these complexes were also able to silenced specific gene. When Hela cells were treated with Rb10-complexed with Akt1-specific siRNA, a evident (at least 50%) reduction of endogenous Akt1 protein expression, compared with treatment with Rb10-complexed with nonspecific siRNA. In contrast, Rb5-complexed with Akt1-siRNA was not able to silence the Akt 1expression.

Rb10 and Rb10big were tested also with the prostate cancer -derived cell line PC3 and were able to efficiently silence Akt1 starting from 24 hours to 72 hours after transfection. Rb10 and Rb10 big are also able to deliver into cells pooled siRNA targeting the Rab5 protein.

Naturally, while the principle of the invention remains the same, the details of construction and the embodiments may widely vary with respect to what has been described and illustrated purely by way of example, without departing from the scope of the present invention.

### REFERENCES

[1] F. Danusso, P. Ferruti: "Synthesis of tertiary amine polymers", (1970) Polymer 11, 88-113]
[2] P. Ferruti, M.A. Marchisio, R. Barbucci (1985), "Synthesis physico-chemical properties and biomedical applications of poly(amido-amine)s", Polymer 26, 1336-1348
[3] P. Ferruti (1996), "Ion-Chelating Polymers (Medical Application)", in: Polymeric Materials Encyclopaedia, vol. 5, J. C. Salamone, Ed. CRC Press INC, Boca Raton, Florida 3334-3359.
[4] P. Ferruti, M.A. Marchisio, R. Duncan (2002), "Poly(amido-amine)s: biomedical applications" Macromol. Rapid. Commun., 23, 332-355.
[5] P. Ferruti, J. Franchini, M. Bencini, E. Ranucci, G. P. Zara, L. Serpe, L. Primo, R. Cavalli, "Prevailingly cationic agmatine-based amphoteric polyamidoamine as a nontoxic, nonhemolytic, and "stealthlike" DNA complexing agent and transfection promoter" (2007) Biomacromolecules 8, 1438-1504]
[6] P. Ferruti, E. Ranucci, F. Trotta, E. Gianasi, G. E. Evagorou, M. Wasil, G. Wilson, R. Duncan, "Synthesis, characterisation and antitumour activity of platinum(II) complexes of novel functionalised poly(amidoamine)s" Macromol. Chem. Phys., 200 (1999) 1644-1654.
[7] A. Sze, D. Erickson, L. Ren, and D. Li, "Zeta-potential measurement using the Smoluchowski equation and the slope of the current-time relationship in electroosmotic flow" J. of Colloid and Interface Sci., 261, (2003) 402-410.
[8] D.J. Gary, N. Puri, Y. Won (2007) "Polymer-based siRNA delivery: Perspectives on the fundamental and Phenomenological distinctions from polymer-based DNA delivery", J. Contr. Rel., 121, 64-73.

## Claims

1. Use of an agmatine-based poly(amidoamine) polymer of
formula (I) 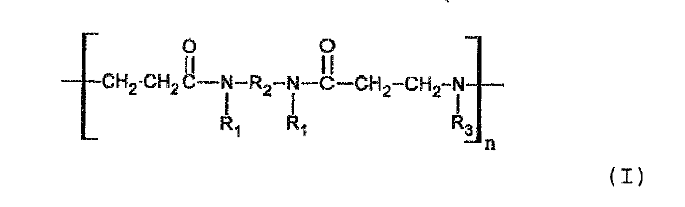 wherein
n is an integer in the range between 20 and 70;
R¹ is H, C₁₋₆ alkyl; or taken together with the N atoms to which is bound and R² is a piperazine ring;
R² is C₂₋₆ alkylen; C₅₋₆ cycloalkylen; CH-COOH, CH-COOR, wherein R is C₁₋₄ alkyl, phenyl or benzyl;
R³ is -(CH₂)ₚ-HN-C(NH₂)=NH,
p is an integer from 1 to 6,
wherein said poly(amidoamine) polymer has a number average molecular weight in the range between 3300 and 33000 and a polydispersity index in the range between 1.25 and 1.75 as an *in vitro* and/or *in vivo* delivery system of at least one RNA molecule into a cell.

2. Use according to claim 1, wherein said poly(amidoamine) polymer has preferably a number average molecular weight in the range between 6,000 and 22,000.

3. Use according to claim 1 or claim 2, wherein R₁ is H, R₂ is CH-COOH and p equals to 4.

4. Use according to claim 1 or claim 2, wherein R₁ is H, R₂ is CH-COOH and p equals to 3.

5. Use according to claim 1 or claim 2, wherein R₁ is H, R₂ is CH-COOH and p equals to 2.

6. Use according to claim 1 or claim 2, wherein R₁ is H, R₂ is CH-COOH and p equals to 5.

7. Use according to claim 1 or claim 2, wherein R₁ is H, R₂ is CH-COOH and p equals to 6.

8. Use according to any one of the preceding claims, wherein said poly(amidoamine) polymer complexes said at least one nucleic acid molecule forming nanoparticles having a size lower than 150 nm, preferably lower than 100 nm.

9. Use according to any one of the preceding claims, wherein said poly(amidoamine) polymer complexes said at least one nucleic acid molecule forming nanoparticles having a size higher than 20 nm, preferably higher than 50 nm.

10. Use according to any one of the preceding claims, wherein said poly(amidoamine) polymer complexes said at least one RNA molecule forming nanoparticles having a size comprised between 50 and 100 nm.

11. Use according to any one of the preceding claims, wherein said poly(amidoamine) polymer complexes said at least one RNA molecule in a RNA molecule:polymer weight ratio in the range between 1:10 and 1:50.

12. Use according to any one of the preceding claims, wherein said poly(amidoamine) polymer complexes said at least one RNA molecule in a RNA molecule:polymer weight ratio in the range between 1:10 and 1:40.

13. Use according to any one of the preceding claims, wherein said poly(amidoamine) polymer has a positive zeta potential in a pH range of 4.0 to 7.4.


SEQUENCE LISTING

<110> Ferruti, Paolo
Manfredi, Amedea
Ranucci, Elisabetta
Cavalli, Roberta
Lembo, David

<120> Non-viral nucleic acid molecules delivery systems

<130> BIT13090-CF

<160> 1

<170> PatentIn version 3.5

<210> 1
<211> 19
<212> RNA
<213> artificial

<220>
<223> AKT-1 siRNA

<400> 1
gcgugaccau gaacgaguu 19